# EUROPEAN PATENT SPECIFICATION

(11) **EP 3 968 884 B1**
(45) Date of publication and mention of the grant of the patent: **25.11.2026**
(21) Application number: 20834729.4
(22) Date of filing: 02.07.2020
(51) Int. Cl.: A61B 18/14, A61B 17/28, A61B 17/285, A61B 17/29, A61B 17/295

(54) **REMOVABLE TIP FOR USE WITH ELECTROSURGICAL DEVICES**
ABNEHMBARE SPITZE ZUR VERWENDUNG MIT ELEKTROCHIRURGISCHEN VORRICHTUNGEN
POINTE AMOVIBLE UTILISABLE AVEC DES DISPOSITIFS ÉLECTROCHIRURGICAUX

(30) Priority: 02.07.2019 US 201916460048; 10.10.2019 US 201916598860
(43) Date of publication of application: 23.03.2022
(73) Proprietor: Alexander, Jamison, Denison TX 75020 (US)
(72) Inventor: Alexander, Jamison, Denison TX 75020 (US)
(74) Representative: Swindell & Pearson Limited
(86) International application number: PCT/US2020/040599
(87) International publication number: WO 2021/003332

(56) References cited:
- WO-A2-2009/036346
- US-A- 5 352 235
- US-A- 5 352 235
- US-A- 5 810 879
- US-A1- 2008 004 656
- US-A1- 2009 209 946
- US-A1- 2012 083 778
- US-A1- 2013 041 292
- US-A1- 2014 135 757

## Description

### TECHNICAL FIELD

This application is directed, in general, to surgical tools and, more specifically, to a removable tip which may be used with an electrosurgical tool for, among other things, cutting, clamping, and cauterizing tissue and vessels.

### BACKGROUND

Electrosurgical devices use high-frequency electrical current to cut, coagulate, and cauterize tissue and vessels and are used in a wide array of surgical procedures. Electrosurgical devices are generally handheld devices guided by a surgeon, making it possible to apply a high-frequency energy to tissue. Electrosurgical devices may be used, for example, to cut tissue, or in other examples, to seal or cauterize tissue and vessels. The energy level may be adjusted by the surgeon by means of control switches disposed in some embodiments on the handheld device, such as, *e.g.* one or more buttons.

During an operation, the surgeon generally has to navigate through several layers of tissue and vessels and may need to cut some tissue or vessels, while sealing or cauterizing other tissue or vessels. For example, electrosurgical devices are commonly used during surgery in order to stop bleeding by using an alternating current directly to heat tissue and thereby reduce blood loss and/or improve surgical vision. In some procedures, there may be a limited amount of space available for surgical instruments. In some embodiments, additional forceps or instruments may be necessary to clamp tissue or clamp around a vessel in order to use an electrosurgical device thereon or navigate around the tissue or vessel due to its size and/or location. In some cases, the surgeon may need additional space within the surgical field or may require an additional hand or set of hands to maneuver the instruments. Such interruptions in the operation may add additional time for the procedure and pose additional risks to the patient.

US 5,352,235 A discloses an insulated laparoscopic grasper with a removable shaft.

US 5,810,879 A discloses a laparoscopic instrument.

Removable tips for electrosurgical instruments are known from US 2013/041292 A1 or US 2012/083778 A1 or US 2008/004656 A1.

### BRIEF DESCRIPTION

Reference is now made to the following descriptions taken in conjunction with the accompanying drawings, in which:
FIG. 1 is a perspective view of one embodiment of a removable tip for use with an electrosurgical device according to the present disclosure;
FIG. 2A is a perspective view of one embodiment of an electrosurgical tool according to the present disclosure, shown with a removable tip in a closed configuration;
FIG. 2B is a perspective view of the electrosurgical tool of FIG. 2A shown with the removable tip in a starting to open configuration;
FIG. 2C is a perspective view of the electrosurgical tool of FIG. 2A shown with the removable tip in a partially open configuration;
FIG. 2D is a perspective view of the electrosurgical tool of FIG. 2A shown with the removable tip in a fully open configuration;
FIG. 3 is a partial sectional view of another embodiment of a removable tip for use with an electrosurgical device according to the present disclosure;
FIG. 4A is a perspective view of one embodiment of an electrosurgical tool according to the present disclosure, shown with a removable tip in a closed configuration;
FIG. 4B is a perspective view of the electrosurgical tool of FIG. 4A shown with the removable tip in a partially open configuration;
FIG. 4C is a perspective view of the electrosurgical tool of FIG. 4A shown with the removable tip in an open configuration;
FIG. 5A is a perspective view of another embodiment of a removable tip for use with an electrosurgical device according to the present disclosure, shown with the removable tip in a closed configuration;
FIG. 5B is a perspective view of the removable tip shown in FIG. 5A, shown with the removable tip in an open configuration;
FIG. 6A is a perspective view of yet another embodiment of a removable tip for use with an electrosurgical device according to the present disclosure, shown with the removable tip in a closed configuration;
FIG. 6B is a perspective view of the removable tip shown in FIG. 6A, shown with the removable tip in an open configuration;
FIG. 6C is an environmental view of the removable tip shown in FIG. 6A and 6B, shown with the removable tip in an open configuration;
FIG. 7A is a perspective view of another embodiment of a removable tip for use with an electrosurgical device according to the present disclosure, shown with the removable tip in an open configuration;
FIG. 7B is a perspective view of the removable tip shown in FIG. 7A, shown with the removable tip in a closed configuration; and
FIG. 7C is an environmental view of the removable tip shown in FIG. 7A and 7B, shown with the removable tip in a closed configuration.

### DETAILED DESCRIPTION

Two primary types of electrosurgical device are known, namely bipolar and monopolar devices. In monopolar arrangements the electrosurgical device is provided with an active electrode and a return electrode is attached to the patient. The electric current flows from the active electrode into the body and returns through the return electrode (which is connected to a grounding circuit). The current density decreases rapidly with distance away from the electrode such that the heating of tissue is localized to the tip of the electrosurgical device. In bipolar devices, a pair of electrodes, for example the tips of forceps, are each connected to the supply circuit and no return electrode is required. When tissue is engaged by or proximal to the pair of electrodes, the high frequency electric current flows through the device and tissue providing a localized heating of the tissue.

Conventional electrosurgical devices may include a single member tip or may include a forceps design which is substantially based upon traditional surgical forceps. As such, a surgeon may have to change electrosurgical instruments, from a single tip to a forceps type design, depending on the function required during a procedure. As such, switching between conventional tips and forceps may add time and risk to a patient during a surgical procedure. In addition, conventional electrosurgical devices are not adjustable to adapt to various body cavity areas or depths and also have the ability to cut, clamp, and cauterize both tissue and vessels.

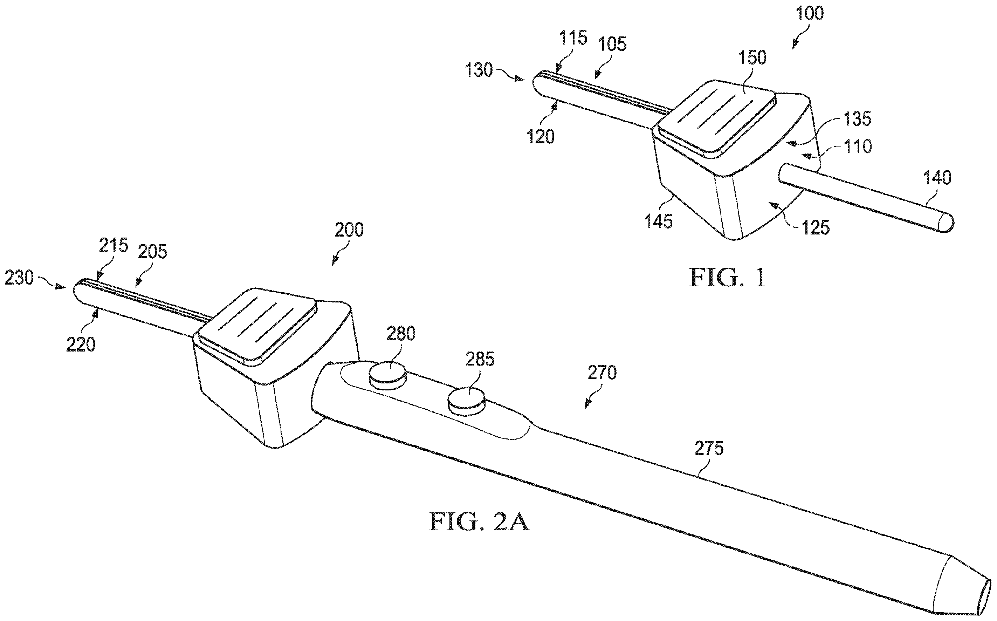

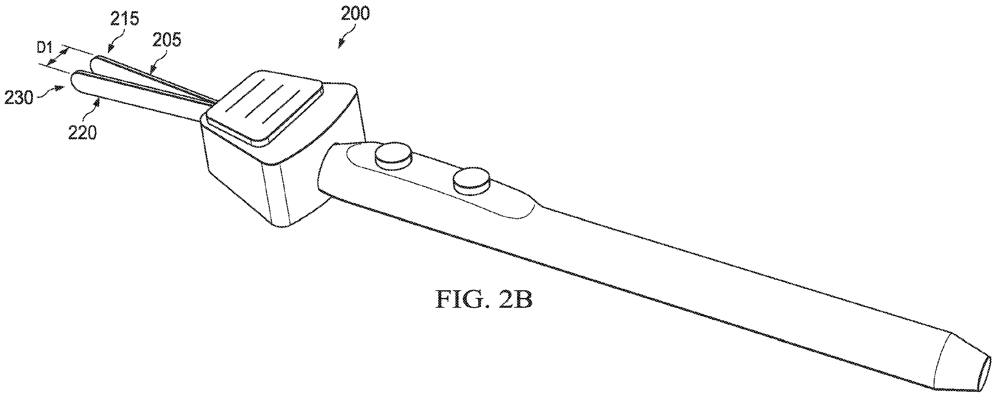

Disclosed herein are embodiments of a removable tip for use with an electrosurgical tool which may be used in surgical procedures. Referring to the drawings, and more specifically, FIG. 1, there is shown a removable tip 100 representing one embodiment of the present disclosure. The removable tip 100 comprises a first tip member 105 having a proximal end 110 and a distal end 115. A second tip member 120 likewise has a proximal end 125 and a distal end 130. In some embodiments, at least one or both of the first tip member 105 and the second tip member 120 may include at least one electrically conductive surface near the distal ends 115 and 130 thereof. The first tip member 105 and second tip member 120 are configured to move from a closed position (as shown in FIG. 1 and FIG. 2A) where the distal end 115 of the first tip member 105 and the distal end 130 of the second tip 120 member are proximate one another, and an open position where the distal end 115 of the first tip member 105 and the distal end 130 of the second tip member 120 are separated by a distance from one another. (Varying stages of open positions will be illustrated in FIGs. 2B-2D). An actuator 135 is coupled to the first tip member 105 and the second tip member 120 for moving the first and second tip members 105 and 120 between the closed position and one or more open positions.

An insertion member 140 is coupled with the proximal ends 110 and 125 of the first and second tip members 105 and 120, the insertion member 140 for inserting the removable tip 100 within an opening in an electrosurgical tool. The insertion member 140 is rotatable within the opening in the electrosurgical tool such that the removable tip 100 may be adjusted to varying angles with respect to the electrosurgical tool. By enabling adjustment of an angle of the removable tip, a surgeon may be able to better maneuver the removable tip 100 within a body cavity than conventional electrosurgical devices. In some examples, the removable tip 100 may need to be rotated to achieve a better placement in the body cavity while enabling the surgeon's hand to be placed in a better or more comfortable or maneuverable position.

The removable tip 100 includes a housing 145 which, in some embodiments, may be positioned between the distal ends 110 and 130 and the proximal ends 115 and 125 of the first and second tip members 105 and 120. The actuator 135 is positioned at least partially within the housing 145. In other embodiments, the housing 145 may be positioned at least partially over the insertion member 140.

In some embodiments, the actuator 135 may include a switch 150. The switch 150, in embodiments not including a housing 145, may be located in various locations on or about the actuator 135. The switch 150 may be configured to move linearly upon pressure applied by a finger or thumb and move the first and second tip members 105 and 120 between closed and open positions, and various positions in between.

In some embodiments, the actuator 135 may include one or more separating members, which may be positioned, in some embodiments within the housing 145. The one or more separating members may be positioned about or between the first and second tip members 105 and 120 for separating the distal ends 115 and 130 of the first and second tip members 105 and 120 when the surgeon engages the actuator 135, which in some embodiments, may be operation of the switch 150. The separating members may also act to move the distal ends 115 and 130 of the first and second tip members 105 and 120 closer together when the actuator 135 may be engaged again, in some embodiments, moving the switch 150 in a second direction.

The removable tip 100 may be adjusted between a closed position and varying open positions (examples shown in FIGs. 2A-2D) depending on what the surgeon may need to use the removable tip 100 for during a surgical procedure. In some examples, the removable tip 100 may need to be in a closed position, for example, when cutting tissue may be needed. However, in certain procedures, clamping the distal end 115 of the first tip member 105 and the distal end 130 of the second tip 120 about a vessel may be useful rather than using a second instrument to clamp around the vessel before applying current from a conventional electrosurgical tip, for example, for cutting or cauterizing a vessel.

The removable tip 100 may also accommodate varying lengths, both longer and shorter, of first tip members 105 and second tip members 120 such that the removable tip 100 may reach deeper within some body cavities without changing the entire electrosurgical tool, such as previously required with conventional fixed electrosurgical tools. In some embodiments, the first tip member 105 and second tip members 120 may be adjustable and/or extendable to varying lengths. In other embodiments, the insertion member 140 may also be adjustable and/or extended to varying lengths.

Referring now to FIGs. 2A through 2D illustrate another embodiment of a removable tip 200 shown coupled into an electrosurgical tool 270, shown in various stages. FIG. 2A illustrates the removable tip in a closed position, wherein distal ends 215 and 230 of first and second tip members 205 and 220 are proximate one another.

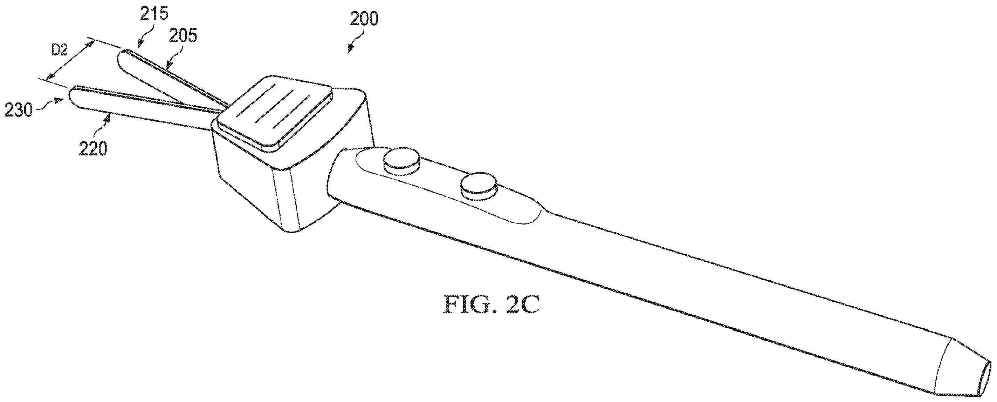

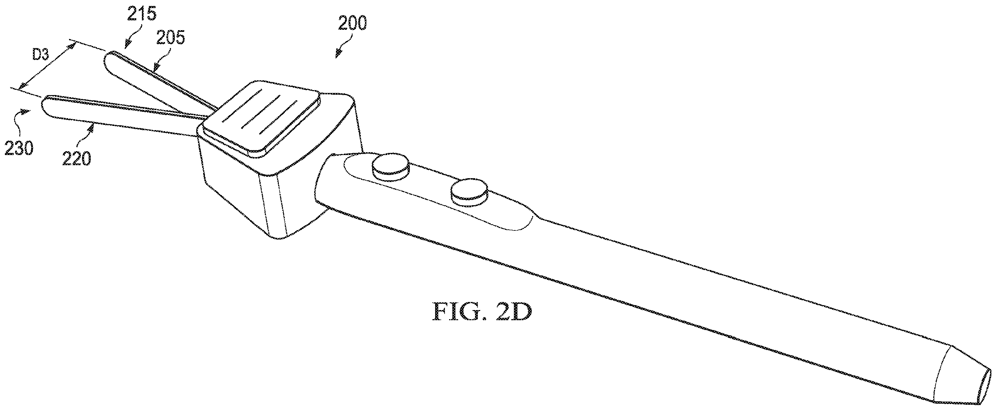

FIGs. 2B through 2D illustrate the removable tip 200 in various open stages, wherein the distal ends 215 and 230 of the first and second tip members 205 and 220 are separated by various distances. FIG. 2B illustrates the removable tip 200 as the first and second tip members 205 and 220 are beginning to open and the distal ends 215 and 230 of the first and second tip members 205 and 220 begin to separate by a first distance D1. FIG. 2C illustrates the removable tip 200 in where the distal ends 215 and 230 first and second tip members 205 and 220 are separate by a second distance D2, which may be about halfway open, wherein halfway open is about 45-55% of fully open. FIG. 2D illustrates the removable tip 200 in a fully open position, where the distal ends 215 and 230 of first and second tip members 205 and 220 are separate by a third distance D3. Fully open, in this disclosure means the distal ends 215 and 230 of the first and second tip members 205 and 220 are within 95-100% open to the maximum functional separation of the distal ends 215 and 230 of the first and second tip members 205 and 220.

Referring again to FIG. 2A, the electrosurgical tool 270 includes a handheld electrosurgical unit 275. The handheld electrosurgical unit 275 may connect with a wall unit, in some embodiments via a plug-in connection such as a wire or cord. The wall unit may supply the electrical current to the electrosurgical tool 270. The first tip member 205 and second tip member 220 may include one or more conductive surfaces for conducting current to tissue to which it may be applied or clasped about. The handheld electrosurgical unit 275 may include, in some embodiments, at least a first button 280 to activate a cutting mode and a second button 285 to activate a cauterizing mode. Additional buttons or functions may be included in some embodiments of the handheld electrosurgical unit 275. Further, some embodiments of the removable tip 200 may be used in monopolar mode while others may be used in bipolar mode.

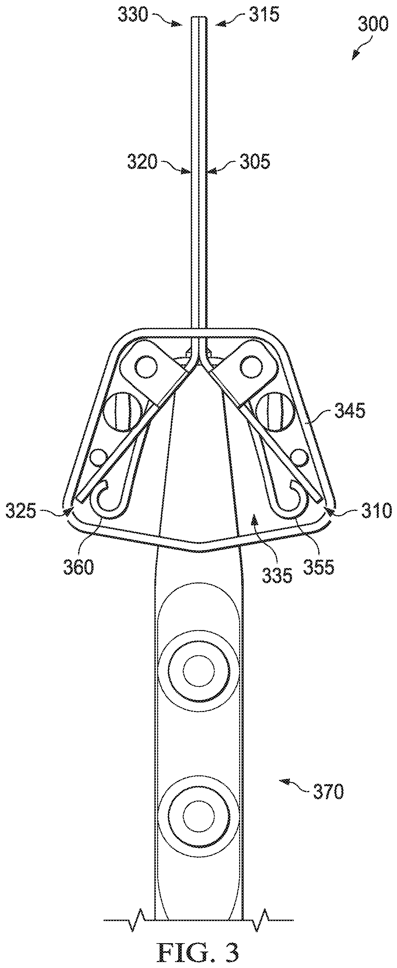

Referring now to FIG. 3, there is shown another embodiment of a removable tip 300 coupled into an electrosurgical tool 370 according to principles of the disclosure. The removable tip 300 includes a first tip member 305 having a proximal end 310 and a distal end 315. A second tip member 320 likewise has a proximal end 325 and a distal end 330. In some embodiments, at least one or both of the first tip member 305 and the second tip member 320 may include at least one electrically conductive surface near the distal ends 315 and 330 thereof. The first tip member 305 and second tip member 320 are configured in some embodiments to move from a closed position to an open position, as discussed hereinabove and shown in FIG.1 through FIG. 2D.

An actuator 335 is coupled to the first tip member 305 and the second tip member 320 for moving the first and second tip members 305 and 320 between the closed position and one or more open positions. The actuator 335 may, in some embodiments, be positioned within a housing 345. The view of FIG. 3 is a partial section-view, showing the inside of housing 345.

In some embodiments, the actuator 335 may include a first separating member 355 and a second separating member 360. In this embodiment, the first separating member 355 is adjacent the proximal end 310 of the first tip member 305 and the second separating member 360 is positioned adjacent the proximal end 325 of the second tip member 320, but the first separating member 355 and second separating member 360 may be positioned between the first and second tip members 305 and 320 at any location that enables the actuator to move the removable tip 300 between closed position and an open position. In another embodiment, the first separating member 355 and the second separating member 360 may be positioned at or incorporating into opposing ends of a single separating component.

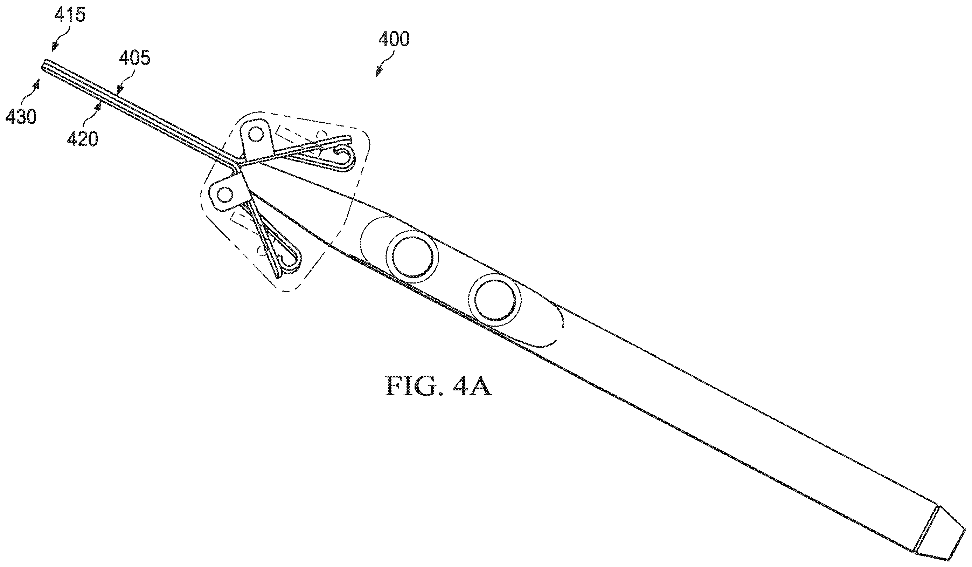

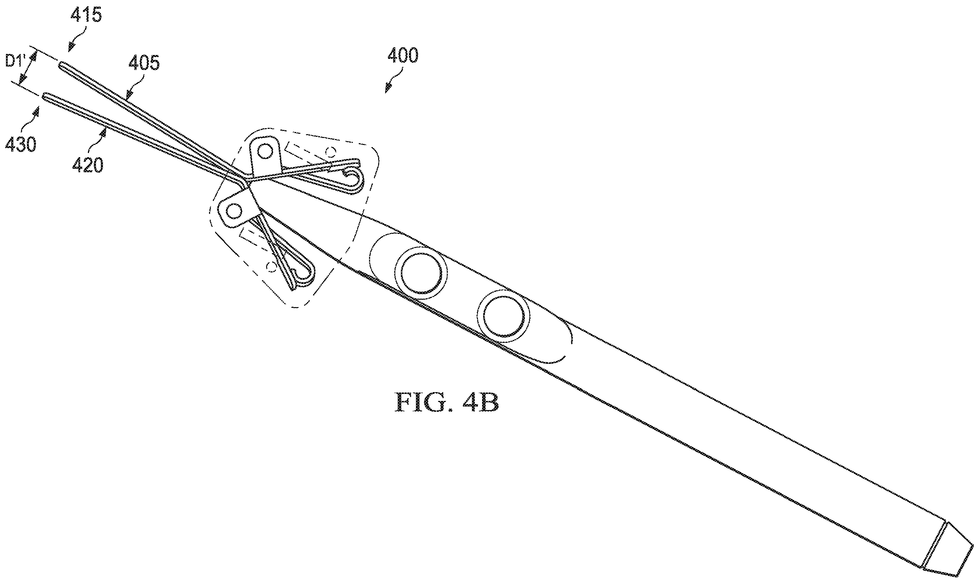

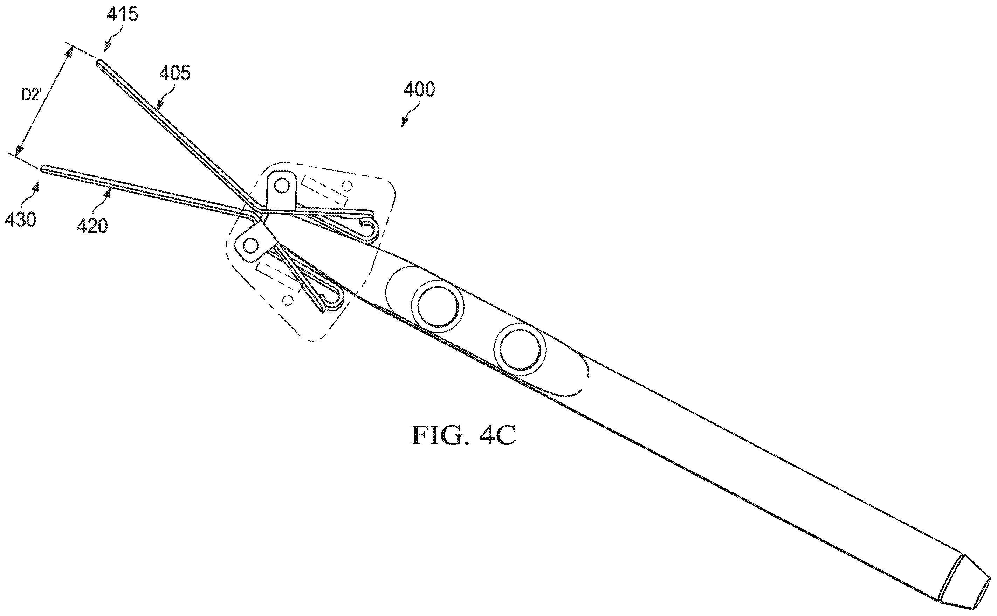

Referring now to FIGs. 4A-4C, there is shown another embodiment of a removable tip 400 shown in 3 different positions: FIG. 4A shows the removable tip 400 in a closed position, wherein a distal end 415 of a first tip member 405 is proximate a distal end 430 of a second tip member 420. FIG. 4B illustrates the removable tip 400 in an opening position, wherein the distal ends 415 and 430 are separate by a distance D1'. FIG. 4C illustrates the removable tip 400 in an open position, wherein the distal ends 415 and 430 are separate by a distance D2'.

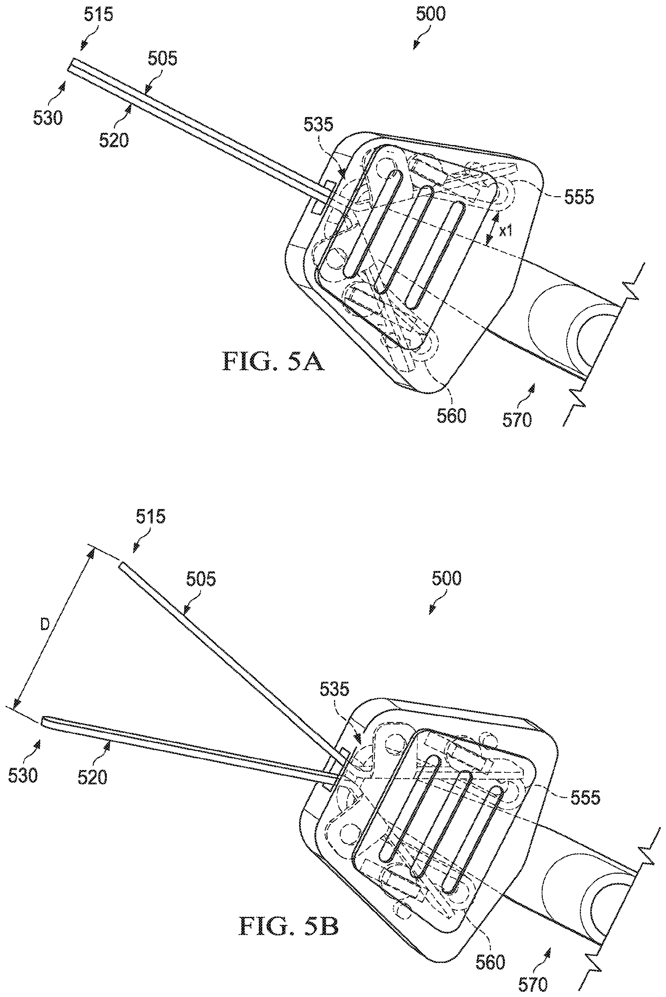

Referring now to FIGs. 5A and 5B there is shown another embodiment of a removable tip 500 shown in a closed position in FIG. 5A and an open position in FIG. 5B. As shown in FIG. 5A, in this embodiment, when the removable tip 500 is closed, a first separating member 555 and a second separating member 560 of an actuator 535 are each positioned at a distance x1 away from a distal end of an electrosurgical tool 570 onto which the removable tip 500 may be coupled. When the removable tip 500 is in an open position, the first and second separating members 555 and 560 may be proximate the distal end of the electrosurgical tool 570 such that there is little or no distance between the first and second separating members 555 and 560 and the distal end of the electrosurgical tool 570. When the removable tip 500 is in varying open positions, similar to those illustrated in FIG. 2B-2D, the distance between the first and second separating members 555 and 560 and the distal end of the electrosurgical tool 570 may vary accordingly.

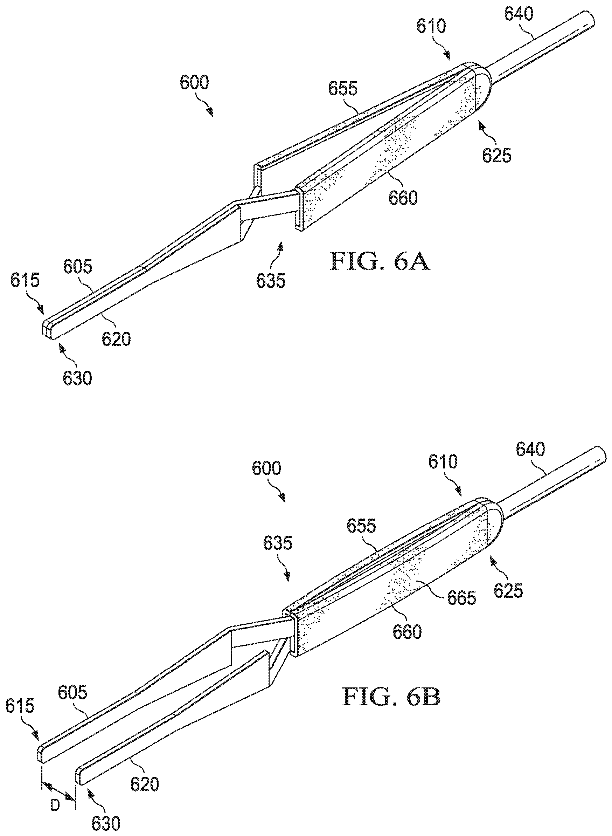

Referring now to FIGS. 6A-6C, there is shown yet another embodiment of a removable tip 600 coupled into a handheld electrosurgical unit 675 according to principles of the disclosure. The removable tip 600 includes a first tip member 605 having a proximal end 610 and a distal end 615. A second tip member 620 likewise has a proximal end 625 and a distal end 630. In some embodiments, at least one or both of the first tip member 605 and the second tip member 620 may include at least one electrically conductive surface at or near the distal ends 615 and 630 thereof. The first tip member 605 and second tip member 620 are configured to move from a closed position, as shown in FIG. 6A to an open position, as shown in FIG. 6B.

An insertion member 640 is coupled with the proximal ends 610 and 625 of the first and second tip members 605 and 620. The insertion member 640 may be for inserting the removable tip 600 within an opening in the handheld electrosurgical unit 675. In some embodiments, the insertion member 640 may be rotatable within the opening in the electrosurgical unit 675 such that the removable tip 600 may be adjusted to varying angles with respect to the electrosurgical tool.

An actuator 635 is coupled to the first tip member 605 and the second tip member 620 for moving the first and second tip members 605 and 620 between the closed position and one or more open positions. In this embodiment, the actuator 635 comprises a portion of the first and second tip members 605 and 620.

In some embodiments, the actuator 635 may include a first separating member 655 and a second separating member 660. In one embodiment, the first separating member 655 and the second separating member 660 each comprise a portion of the first and second tip members 605 and 620. In some embodiments, to actuate the first and second tip members 605 and 610 from a closed position as shown in FIG. 6A to an open position shown in FIG. 6B, axial pressure may be applied to the first separating member 655 and the second separating member 660, moving the first separating member 655 and the second separating member 660 toward each other as the distal ends 615 and 630 move apart from one another. In some examples, the pressure may be applied to at least exterior surfaces of the first and second separating members 655 and 660.

In some embodiments, at least a portion of the first and second tip members 605 and 620 may be removable and/or replaceable. In one embodiment, the distal ends 615 and 630 may be removed at or above the first separating member 655 and the second separating member 660. The distal ends 605 and 620 may couple with the first separating member 655 and the second separating member 660 by a snap fit, tension fit, a locking fit, and other traditional coupling means for removing and replacing a distal end of a longitudinal member such as the first and second tip members 605 and 620.

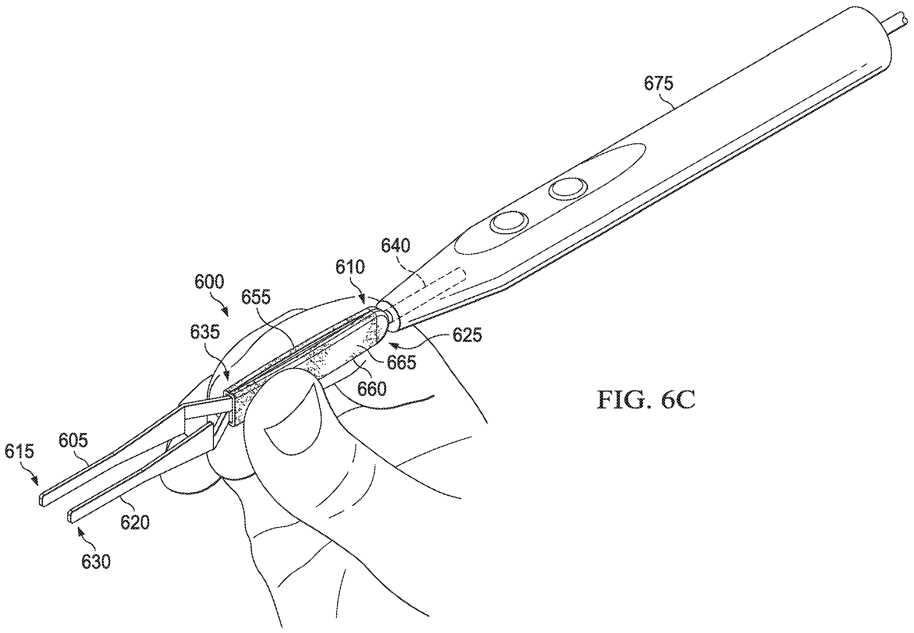

Referring to FIG. 6C, there is shown an environmental view of the removable tip 600, shown with the actuator 635 engaged such that the distal ends 615 and 630 of the first and second tip members 605 and 620 are opened apart from each other. In this embodiment, the actuator 635 is shown engaged by fingers applying axial pressure to the first and second separating members 655 and 660. In one embodiment, the first separating member 655 and second separating member 660 may include a coating or covering 665 on at least an exterior surface of each, but in some embodiments, the coating or covering may cover or coat most or all surfaces of the first and second separating members 655 and 660. The coating 665 may comprise polymers, rubbers, plastics, and any other suitable material which may provide, in some embodiments, insulation from any electrical current running through the removable tip 600 and may also, in other embodiments, provide a non-slip grip, and/or comfort grip to a surgeon handling the removable tip 600.

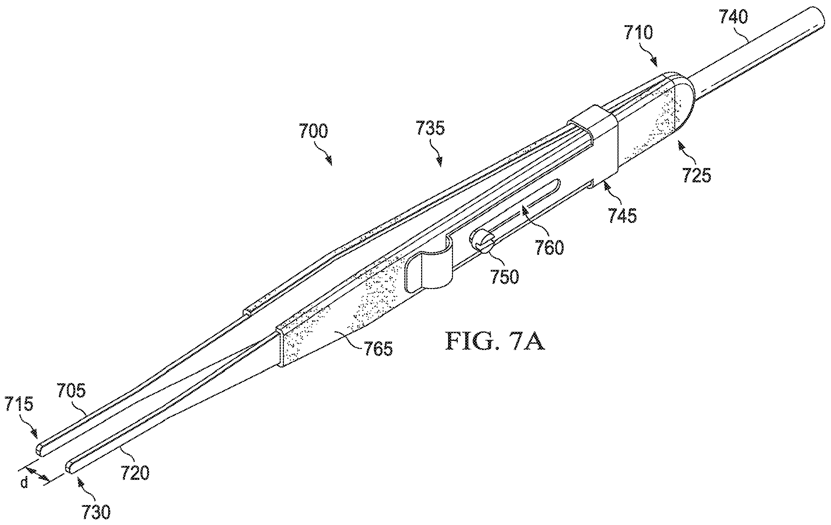

Referring now to FIGs. 7A-7C, there is shown yet another embodiment of a removable tip 700 coupled into a handheld electrosurgical unit 775 according to principles of the disclosure. The removable tip 700 includes a first tip member 705 having a proximal end 710 and a distal end 715. A second tip member 720 likewise has a proximal end 725 and a distal end 730. In some embodiments, at least one or both of the first tip member 705 and the second tip member 720 may include at least one electrically conductive surface near the distal ends 715 and 730 thereof. The first tip member 705 and second tip member 720 are configured to move from a closed position to an open position, as discussed hereinabove and shown in FIG. 7A-7C. An insertion member 740 extends from the removable tip near the proximal ends 710 and 725. The insertion member 740 may be for inserting the removable tip 700 within an opening in the handheld electrosurgical unit 775.

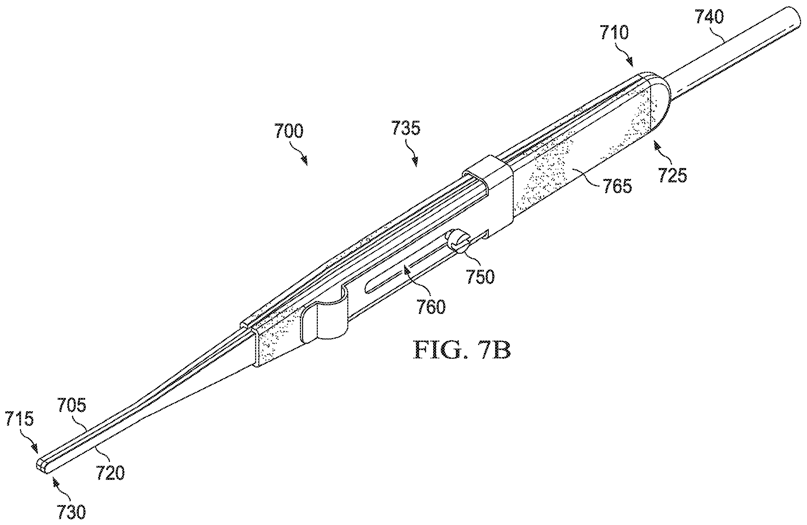

The removable tip includes an actuator 735 for moving the distal ends 715 and 730 of the first and second tip members 705 and 720 between a closed position and one or more open positions. In one embodiment, the actuator 735 may comprise part of the first tip member 705 and the second tip member 720. In one embodiment, the actuator 735 may include a locking mechanism 745, which in one embodiment includes a locking pin 750 positioned within a lateral slot 760. In some embodiments, the locking mechanism may be coupled around both the first and second tip members 705 and 720, and in some embodiments, near the proximal ends 710 and 725 thereof. In one embodiment, the first and second tip members 705 and 720 are in an "open position" as shown in FIG. 7A, which the distal ends 715 and 730 are separated by a distance d. To move the distal ends 715 and 730 toward a "closed position" a shown in FIG. 7B, axial pressure may be applied to the first and second tip members 705 and 720 and the locking mechanism 745 may be moved toward the distal ends 715 and 730. Once the distal ends 715 and 730 are closed together, in some embodiments, the pin 750 may be engaged to maintain the locking mechanism in a locked position. The pin 750 may be engaged by turning the pin 750 to a locked position, or in some embodiments may be locked in a compressed position.

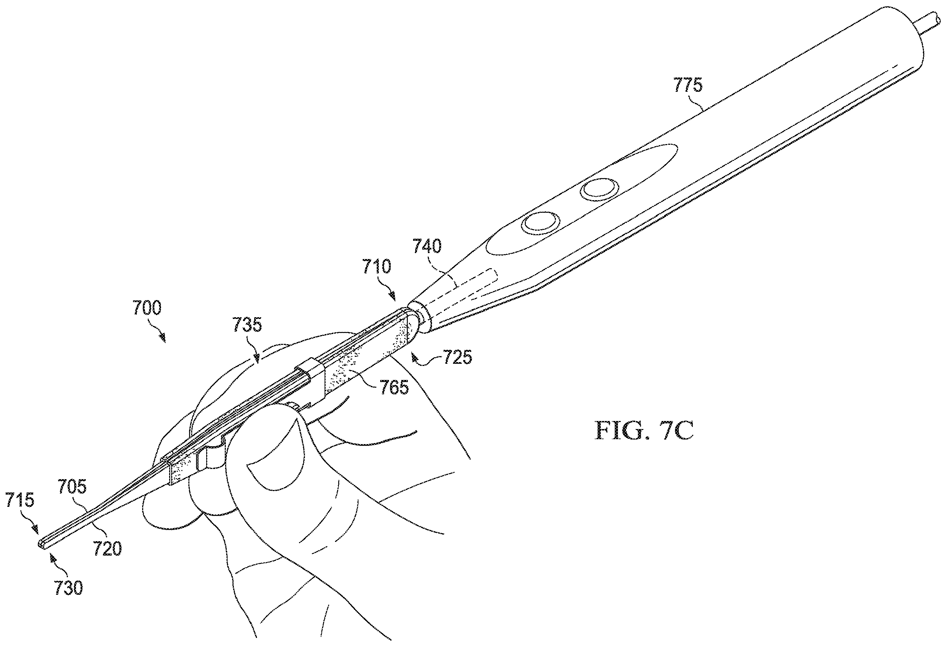

Referring to FIG. 7C, there is shown an environmental view of the removable tip 700, shown with the actuator 735 engaged such that the distal ends 715 and 730 of the first and second tip members 705 and 720 are closed proximal each other. In this example, the actuator may include a coating or covering 765 at least on an outer surface. The coating 765 may comprise polymers, rubbers, plastics, and any other suitable material which may provide, in some embodiments, insulation from any electrical current running through the removable tip 700 and may also, in other embodiments, provide a non-slip grip, and/or comfort grip to a surgeon handling the removable tip 700.

In some embodiments, all of part of the removable tip 700 may include a non-stick coating which may be placed, in some embodiments, on at least the distal ends 715 and 730 of the first and second tip members 705 and 720 to reduce build-up of charred tissue thereon and also reduce a risk of thermal injuries. Several different types of non-stick coatings have been used or suggested for application to electrosurgical electrodes. Some of the different non-stick coatings or materials include fluorinated hydrocarbon materials, polytetrafluoroethylene (PTFE), perfluoroalkoxy, flexible silicone elastomers, ceramic composites, paralyene siloxane polymers, epoxy modified rigid silicone powder, silicone resin, and other suitable non-stick coatings.

The invention is defined in the appended claims.

## Claims

1. A removable tip (100; 200; 300; 400; 500; 600; 700) for use with an electrosurgical tool (270; 370; 570), the removable tip comprising:
a housing (145; 345);
a tip extending from within the housing, the tip including a first tip member (105; 205; 305; 405; 605; 705) having a proximal end (110; 310; 610; 710) and a distal end (115; 215; 315; 415; 615; 715), and a second tip member (120; 220; 320; 420; 620; 720) having a proximal end (125; 325; 625; 725) and a distal end (130; 230; 330; 430; 630; 730), wherein the proximal ends of the first and second tip members are directly coupled within the housing;
an actuator (135; 335; 535; 635; 735) positioned at least partially within the housing, the actuator directly coupled to the first tip member and the second tip member, the actuator for moving the first and second tip members between a closed position where the distal end of the first tip member and the distal end of the second tip member are proximate one another, and an open position where the distal end of the first tip member and the distal end of the second tip member are separated by a distance from one another; and
an insertion member (140; 640; 740) coupled with the proximal ends of the first and second tip members, the insertion member for inserting the removable tip within an opening in the electrosurgical tool, **characterized in that** the insertion member, once fully inserted and positioned into the electrosurgical tool, is rotatable within the opening in the electrosurgical tool.

2. The electrosurgical tool according to Claim 1, wherein the housing is positioned at least partially over the insertion member.

3. The electrosurgical tool according to Claim 1, wherein the actuator is positioned at least partially within the housing and includes a switch (150), the switch configured to move linearly by pressure applied by a finger or thumb and move the first and second tip members between the closed and open positions.

4. The electrosurgical tool according to Claim 1, wherein the actuator includes separating members (355, 360; 555, 560; 655, 660) for separating the first and second tip members when the switch is moved in a first direction wherein, optionally, the actuator includes separating members within the housing for moving the first and second tip members toward each other when the switch is moved in a second direction.

5. The electrosurgical tool according to Claim 1, wherein each of the first and second tip members comprise at least one electrically conductive surface.

6. The electrosurgical tool according to Claim 1, wherein the first and second tip members are adjustable and/or extendable to varying lengths.

7. An electrosurgical tool, the electrosurgical tool comprising:
a handheld electrosurgical unit (275; 675; 775) configured to be connected with an electrosurgical wall unit and including at least a first button (280) to activate a cutting mode and a second button (285) to activate a cauterizing mode; and
a removable tip according to any of the preceding claims.

## Patentansprüche

1. Abnehmbare Spitze (100; 200; 300; 400; 500; 600; 700) zur Verwendung mit einem elektrochirurgischen Werkzeug (270; 370; 570), wobei die abnehmbare Spitze Folgendes umfasst:
ein Gehäuse (145; 345);
eine Spitze, die sich von innerhalb des Gehäuses erstreckt, wobei die Spitze ein erstes Spitzenelement (105; 205; 305; 405; 605; 705), das ein proximales Ende (110; 310; 610; 710) und ein distales Ende (115; 215; 315; 415; 615; 715) aufweist, und ein zweites Spitzenelement (120; 220; 320; 420; 620; 720), das ein proximales Ende (125; 325; 625; 725) und ein distales Ende (130; 230; 330; 430; 630; 730) aufweist, enthält, wobei die proximalen Enden des ersten und des zweiten Spitzenelements direkt innerhalb des Gehäuses gekoppelt sind;
einen Aktuator (135; 335; 535; 635; 735), der mindestens teilweise innerhalb des Gehäuses positioniert ist, wobei der Aktuator direkt an das erste Spitzenelement und das zweite Spitzenelement gekoppelt ist, wobei der Aktuator zum Bewegen des ersten und des zweiten Spitzenelements zwischen einer geschlossenen Position, in der das distale Ende des ersten Spitzenelements und das distale Ende des zweiten Spitzenelements nahe zueinander liegen, und einer offenen Position, in der das distale Ende des ersten Spitzenelements und das distale Ende des zweiten Spitzenelements durch einen Abstand voneinander getrennt sind, dient; und
ein Einführelement (140; 640; 740), das mit den proximalen Enden des ersten und des zweiten Spitzenelements gekoppelt ist, wobei das Einführelement zum Einführen der abnehmbaren Spitze innerhalb einer Öffnung in dem elektrochirurgischen Werkzeug dient, **dadurch gekennzeichnet, dass** das Einführelement, sobald es vollständig in das elektrochirurgische Werkzeug eingeführt und positioniert ist, innerhalb der Öffnung in dem elektrochirurgischen Werkzeug drehbar ist.

2. Elektrochirurgisches Werkzeug nach Anspruch 1, wobei das Gehäuse mindestens teilweise über dem Einführelement positioniert ist.

3. Elektrochirurgisches Werkzeug nach Anspruch 1, wobei der Aktuator mindestens teilweise innerhalb des Gehäuses positioniert ist und einen Schalter (150) enthält, wobei der Schalter dazu konfiguriert ist, sich durch Druck, der durch einen Finger oder Daumen ausgeübt wird, linear zu bewegen und das erste und das zweite Spitzenelement zwischen der geschlossenen und der offenen Position zu bewegen.

4. Elektrochirurgisches Werkzeug nach Anspruch 1, wobei der Aktuator Trennelemente (355, 360; 555, 560; 655, 660), um das erste und das zweite Spitzenelement zu trennen, wenn der Schalter in eine erste Richtung bewegt wird, enthält, wobei der Aktuator optional Trennelemente innerhalb des Gehäuses enthält, um das erste und das zweite Spitzenelement zueinander zu bewegen, wenn der Schalter in eine zweite Richtung bewegt wird.

5. Elektrochirurgisches Werkzeug nach Anspruch 1, wobei jedes von dem ersten und dem zweiten Spitzenelement mindestens eine elektrisch leitende Oberfläche umfasst.

6. Elektrochirurgisches Werkzeug nach Anspruch 1, wobei das erste und das zweite Spitzenelement auf unterschiedliche Längen einstellbar und/oder ausfahrbar sind.

7. Elektrochirurgisches Werkzeug, wobei das elektrochirurgische Werkzeug Folgendes umfasst:
eine elektrochirurgische Handeinheit (275; 675; 775), die dazu konfiguriert ist, mit einer elektrochirurgischen Wandeinheit verbunden zu werden, und mindestens eine erste Taste (280) zum Aktivieren eines Schneidmodus und eine zweite Taste (285) zum Aktivieren eines Kauterisierungsmodus enthält; und
eine abnehmbare Spitze nach einem der vorhergehenden Ansprüche.

## Revendications

1. Pointe amovible (100 ; 200 ; 300 ; 400 ; 500 ; 600 ; 700) utilisable avec un outil électrochirurgical (270 ; 370 ; 570), la pointe amovible comprenant :
un boîtier (145 ; 345) ;
une pointe s'étendant à partir de l'intérieur du boîtier, la pointe comprenant un premier élément de pointe (105 ; 205 ; 305 ; 405 ; 605 ; 705) ayant une extrémité proximale (110 ; 310 ; 610 ; 710) et une extrémité distale (115 ; 215 ; 315 ; 415 ; 615 ; 715), et un second élément de pointe (120 ; 220 ; 320 ; 420 ; 620 ; 720) ayant une extrémité proximale (125 ; 325 ; 625 ; 725) et une extrémité distale (130 ; 230 ; 330 ; 430 ; 630 ; 730), dans lequel les extrémités proximales des premier et second éléments de pointe sont directement couplées à l'intérieur du boîtier ;
un actionneur (135 ; 335 ; 535 ; 635 ; 735) positionné au moins partiellement à l'intérieur du boîtier, l'actionneur étant directement couplé au premier élément de pointe et au second élément de pointe, l'actionneur permettant de déplacer les premier et second éléments de pointe entre une position fermée où l'extrémité distale du premier élément de pointe et l'extrémité distale du second élément de pointe sont proches l'une de l'autre, et une position ouverte où l'extrémité distale du premier élément de pointe et l'extrémité distale du second élément de pointe sont séparées par une distance l'une de l'autre ; et
un élément d'insertion (140 ; 640 ; 740) couplé aux extrémités proximales des premier et second éléments de pointe, l'élément d'insertion permettant d'insérer la pointe amovible à l'intérieur d'une ouverture dans l'outil électrochirurgical, **caractérisé en ce que** l'élément d'insertion, une fois complètement inséré et positionné dans l'outil électrochirurgical, peut tourner à l'intérieur de l'ouverture dans l'outil électrochirurgical.

2. Outil électrochirurgical selon la revendication 1, dans lequel le boîtier est positionné au moins partiellement au-dessus de l'élément d'insertion.

3. Outil électrochirurgical selon la revendication 1, dans lequel l'actionneur est positionné au moins partiellement à l'intérieur du boîtier et comprend un commutateur (150), le commutateur étant configuré pour se déplacer linéairement par pression appliquée par un doigt ou un pouce et déplacer les premier et second éléments de pointe entre les positions fermée et ouverte.

4. Outil électrochirurgical selon la revendication 1, dans lequel l'actionneur comprend des éléments de séparation (355, 360 ; 555, 560 ; 655, 660) permettant de séparer les premier et second éléments de pointe lorsque le commutateur est déplacé dans une première direction, dans lequel, éventuellement, l'actionneur comprend des éléments de séparation à l'intérieur du boîtier permettant de déplacer les premier et second éléments de pointe l'un vers l'autre lorsque le commutateur est déplacé dans une seconde direction.

5. Outil électrochirurgical selon la revendication 1, dans lequel chacun des premier et second éléments de pointe comprennent au moins une surface électriquement conductrice.

6. Outil électrochirurgical selon la revendication 1, dans lequel les premier et second éléments de pointe sont réglables et/ou extensibles à des longueurs variables.

7. Outil électrochirurgical, l'outil électrochirurgical comprenant :
une unité électrochirurgicale portative (275 ; 675 ; 775) configurée pour être connectée à une unité de paroi électrochirurgicale et comprenant au moins un premier bouton (280) pour activer un mode de coupe et un deuxième bouton (285) pour activer un mode de cautérisation ; et
une pointe amovible selon l'une quelconque des revendications précédentes.
